# EUROPÄISCHE PATENTSCHRIFT

(11) **EP 4 210 806 B1**
(45) Veröffentlichungstag und Bekanntmachung des Hinweises auf die Patenterteilung: **10.09.2025**
(21) Anmeldenummer: 21769053.6
(22) Anmeldetag: 17.08.2021
(51) Int. Cl.: A61M 39/10, B01D 15/22, A61M 5/31, B01L 3/00, G01N 30/60

(54) **LUER-SÄULE**
LUER COLUMN
COLONNE LUER

(30) Priorität: 07.09.2020 DE 202020105143 U
(43) Veröffentlichungstag der Anmeldung: 19.07.2023
(73) Patentinhaber: Elbatron GmbH, 22926 Ahrensburg (DE)
(72) Erfinder: HERRMANN, Rainer, 22949 Ammersbek (DE)
(74) Vertreter: Patentanwälte Hemmer Lindfeld Frese Partnerschaft mbB
(86) Internationale Anmeldenummer: PCT/DE2021/200112
(87) Internationale Veröffentlichungsnummer: WO 2022/048715

(56) Entgegenhaltungen:
- WO-A1-2011/005801
- WO-A1-2011/069289
- US-A1- 2016 209 393
- US-A1- 2019 107 516

## Beschreibung

Die Erfindung betrifft eine Luer-Säule und ein Luer-Säulen-System.

Bei der labortechnischen Untersuchung von Proben oder bei anderen analytischen Verfahren werden Lösungen oftmals filtriert. Abhängig von den zu filtrierenden Volumina werden spritzenähnliche Filtereinheiten in Form von Luer-Säulen verwendet, bei denen Filter mit Standardformat in Luer-Säulen eingesetzt sind. Luer-Säulen können generell auch zu anderen Zwecken in Laboren eingesetzt werden. Bei bestimmten Vorgängen werden mehrere Luer-Säulen nacheinander durch eine Flüssigkeit durchflossen, beispielsweise um bestimmte Schadstoffe aus Proben lösen. Zum Kaskadieren von Luer-Säulen sind Adapter vorgesehen, die mit einem Luer-Kegel, einer Luer-Säule und einem Einlass einer anderen Luer-Säule verbindbar sind. Die Verwendung von Adaptern bedarf jedoch eines manuellen Einstellungsaufwands und erhöht das Risiko potentieller Leckagen sowie aufgrund der höheren Teilevielfalt die Kosten.

Die US 2019/107516 A1 beschreibt ein Vakuum-Flüssigkeitsextraktionssystem, das mit Luer-Adaptern zur Kaskadierung von Säulen arbeitet. Die WO 2011/069289 A1 beschreibt ein System zur visuellen Fluoreszenzchromatographie, das ebenfalls mit einem Luer-Adapter arbeitet. Die US 2016/0209393 A1 beschreibt ein Gerät zur Extraktion eines Analyts aus einer flüssigen Probe mit einer distalseitig abgestuften Säule, um ein Extraktionsmedium innenseitig aufzunehmen. Die WO 2011/005801 A1 beschreibt eine Dichtungsmatte für eine Mehrzahl an Luer-Säulen.

Aufgabe der vorliegenden Erfindung ist es, eine alternative Luer-Säule bereitzustellen, mit der die labortechnische Handhabung vereinfacht und eine einfachere, verbesserte Verbindung mehrerer Luer-Säulen zur Kaskadierung erreicht wird.

Diese Aufgabe wird gemäß der Erfindung durch eine Luer-Säule mit den in Anspruch 1 angegebenen Merkmalen gelöst. Vorteilhafte Ausgestaltungen der Luer-Säule ergeben sich aus den Unteransprüchen, der nachfolgenden Beschreibung und den Zeichnungen, wobei die in den Unteransprüchen und der Beschreibung angegebenen Merkmale jeweils für sich aber auch in geeigneter Kombination anwendbar sind.

Es wird eine Luer-Säule mit einem Einlassende, einem davon beabstandeten Auslassabschnitt und einem sich von dem Einlassende zu dem Auslassabschnitt erstreckenden Gehäusemantel vorgeschlagen, wobei sich der Auslassabschnitt in eine von dem Einlassende abgewandte Richtung erstreckt und an einem distalen Ende einen männlichen Luer-Kegel ausbildet, wobei ein Übergangsbereich zwischen dem Luer-Kegel und dem Gehäusemantel einen abgestuften Durchmesser mit mindestens zwei Durchmesserstufen mit jeweils einer radialen Ringfläche aufweist.

Die Luer-Säule ist ein Hohlkörper, der weitgehend hohlzylindrisch ausgeformt ist. Der Gehäusemantel, der sich von den Einlassende zu dem Auslassabschnitt erstreckt, läuft von dem Einlassende zu dem Auslassabschnitt konisch sehr leicht zu. Beispielhaft könnte ein Einstellwinkel deutlich unter 1° liegen. Die Luer-Säule bzw. der Gehäusemantel können aus einem Kunststoff hergestellt sein, insbesondere einem zumindest teilweise transparenten Kunststoff, beispielsweise Polypropylen oder Polyethylen.

Der Gehäusemantel umschließt einen Hohlraum, der sich vollständig von dem Einlassende zu dem Auslassabschnitt in den Luer-Kegel hinein erstreckt. Dieser Hohlraum könnte beispielsweise dazu vorgesehen sein, ein Filterelement aufzunehmen. Ein Fluid, welches durch das Einlassende in den Hohlraum gerät, könnte dann schwerkraftbedingt oder durch Wirkung eines in dem Hohlraum bewegten Kolbens durch das Filterelement zu dem Auslassabschnitt strömen und an dessen distalem Ende austreten. Die Luer-Säule kann durch Integration eines Filter-Einsatzes eine Filtereinheit bilden.

Das distale Ende weist einen männlichen Luer-Kegel auf, der von einem weiblichen Luer-Innenkegel umgeben werden kann, um eine Luer-Verbindung herzustellen. Nach den gängigen Spezifikationen könnte der Luer-Kegel eine Länge von höchstens 7,5 mm sowie einen bestimmten Kegelwinkel aufweisen. Damit wird sichergestellt, dass eine temporäre Verbindung mit einem anderen, von einem Fluid durchströmbaren Element hergestellt werden kann.

Eine Besonderheit der erfindungsgemäßen Luer-Säule liegt in der weiteren Ausgestaltung des Auslassabschnitts. In dem Übergangsbereich zwischen dem Luer-Kegel und dem Gehäusemantel ist ein abgestufter Durchmesser mit mindestens zwei Durchmesserstufen mit jeweils einer radialen Ringfläche vorgesehen. Dies bedeutet, dass der Übergang zwischen dem Luer-Kegel und dem Ende eines zylindrischen Teils des Gehäusemantels nicht kontinuierlich aufweitend bzw. verjüngend ist, sondern abgestuft. Jeder Abstufung ist ein bestimmter, von dem Luer-Kegel aus betrachtet, stufenweise vergrößerter Durchmesser zugeordnet und wird durch eine radiale Ringfläche begrenzt. Die radiale Ringfläche weist jeweils eine axiale Länge auf, die einen Teil der axialen Erstreckung des Auslassabschnitts bildet. Die radiale Ringfläche ist bevorzugt symmetrisch zu einer Längsachse der Luer-Säule, welche mittig durch das Einlassende und den Luer-Kegel verläuft, ausgebildet. Bevorzugt ist jede radiale Ringfläche in ihrer Länge begrenzt und dafür vorgesehen, in das Einlassende einer anderen Luer-Säule gesteckt zu werden, dessen Innendurchmesser mit dem Außendurchmesser der jeweiligen Durchmesserstufe weitgehend übereinstimmt.

Es ist damit möglich, Luer-Säulen ohne Adapter zu verwenden, um sie mit anderen Luer-Säulen zu verbinden. Es können damit beispielsweise sehr einfach Kaskaden von Luer-Säulen zusammengesteckt und mit individuellen Filtereinsätzen bestückt werden. Die Luer-Säule ist dann nicht auf eine schwerkraftbedingte Durchströmung mit einem Fluid beschränkt, sondern durch einen Kolben oder Ähnliches kann das Fluid aktiv gefördert werden.

Erfindungsgemäß sind zwischen dem Luer-Kegel und dem Gehäusemantel zwei oder mehr Durchmesserstufen angeordnet. Eine erfindungsgemäße Luer-Säule ist demnach dazu angepasst, mit einer Luer-Säule verbunden zu werden, die einen von zwei oder mehr möglichen Durchmessern an dem Einlassende aufweist. Selbstverständlich wäre auch denkbar, den Auslassabschnitt mit drei oder vier Durchmesserstufen auszubilden. Neben der Verbindung mit einem Einlassende einer Luer-Säule ist selbstverständlich auch eine Verbindung mit einem weiblichen Luer-Kegel möglich.

In einer vorteilhaften Ausführungsform weisen die radialen Ringflächen der Durchmesserstufen jeweils dieselbe axiale Länge auf. Die form- und kraftschlüssige Verbindung kann folglich für jede Durchmesserstufe auf die gleiche Weise erreicht werden.

Es ist vorteilhaft, wenn die radialen Ringflächen an ihren axialen Endkanten abgerundet oder abgeschrägt sind. Durch das Abrunden oder Abschrägen wird ein scharfkantiger Übergang insbesondere an den mindestens zwei Durchmesserstufen erreicht. Das Einstecken der betreffenden Durchmesserstufe in ein Einlassende wird dadurch außerdem vereinfacht und eine Beschädigung verhindert. Die Luer-Säule kann auch mehrfach verwendet werden.

Weiterhin könnte die radiale Ringfläche eine axiale Länge von höchstens 7,5 mm, bevorzugt von 3 bis 6 mm und besonders bevorzugt von 4 mm aufweisen. Die radiale Ringfläche kann mit einer solchen Länge eine ausreichende Fixierung an einem Einlassende einer anderen Luer-Säule hervorrufen. Da die Länge jedoch stark begrenzt ist, ist die Verbindung dennoch leicht lösbar.

Bevorzugt weist das Einlassende einen Innendurchmesser auf, der mit einem Außendurchmesser einer der mindestens zwei Durchmesserstufen eine Klemmpassung bildet. Die Klemmpassung kann dadurch erreicht werden, dass der äußere Durchmesser der betreffenden radialen Ringfläche um einen Bruchteil größer ist als der korrespondierende Innendurchmesser des Einlassendes. Der Durchmesserunterschied ist auf das verwendete Material und die gewünschte Größe des Einlassendes abzustimmen. Bei einem thermoplastischen Material, beispielsweise Polypropylen, könnte eine Durchmesserabweichung einen oder wenige Zehntel Millimeter betragen. Die Klemmpassung führt nicht nur zu einer mechanischen Verbindung zum Halten der Luer-Säulen aneinander, sondern auch zu einer Abdichtung zwischen ihnen.

Erfindungsgemäß ist die Luer-Säule einstückig ausgeführt. Eine Abdichtung zwischen der Luer-Säule und einem separaten Luer-Anschluss ist nicht erforderlich.

Die Erfindung betrifft ferner ein Luer-Säulen-System mit mindestens zwei Luer-Säulen gemäß der vorhergehenden Beschreibung, wobei ein Außendurchmesser einer der mindestens zwei Durchmesserstufen einer der Luer-Säulen angepasst ist, eine Klemmpassung zwischen der betreffenden radialen Ringfläche und dem Einlassende der anderen Luer-Säule zu bilden.

Die Erfindung ist nachfolgend anhand von in den Zeichnungen dargestellten Ausführungsbeispielen näher erläutert. Es zeigen:
Figur 1a bis 1d ein erstes Ausführungsbeispiel einer erfindungsgemäßen Luer-Säule und
Figur 2a bis 2d ein zweites Ausführungsbeispiel einer erfindungsgemäßen Luer-Säule.

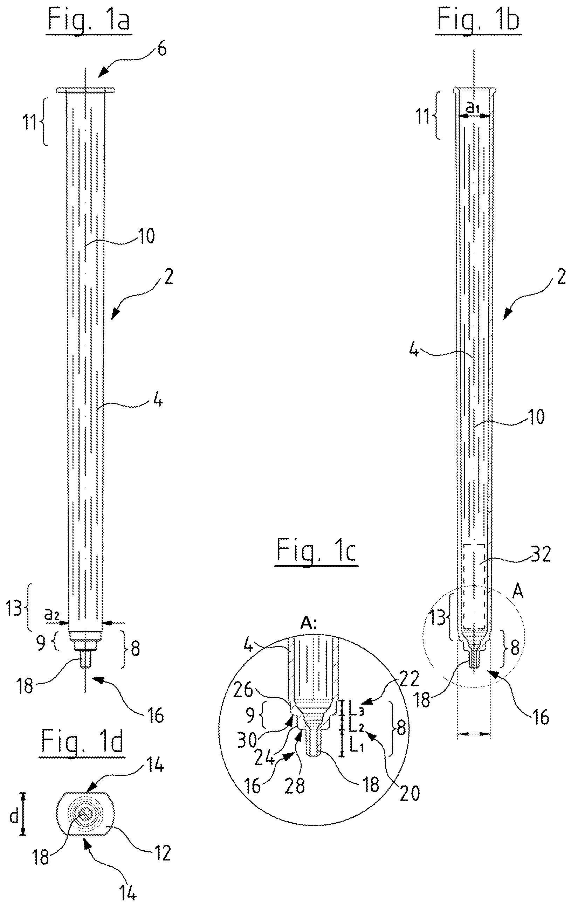

Figur 1a bis 1d zeigen ein Ausführungsbeispiel einer Luer-Säule 2. Diese besitzt einen Gehäusemantel 4, der sich von einem Einlassende 6 zu einem Auslassabschnitt 8 erstreckt und im Wesentlichen eine zylindrische Form besitzt. Der Gehäusemantel 4 kann eine konstante Wandstärke aufweisen. Er bildet eine radiale Umfangsfläche, die weitgehend parallel zu einer Mittelachse 10 der Luer-Säule 2 verläuft. Der Gehäusemantel 4 läuft, wie hier gezeigt, leicht konisch zu und kann beispielhaft einen Winkel von weniger als 1° und beispielsweise 0,25° zu der Mittelachse 10 einschließen. Dadurch kann in einen ersten am Einlassende 6 befindlichen Endabschnitt 11 des Gehäusemantels 4 ein am Auslassabschnitt 8 befindlicher zweiter Endabschnitt 13 des Gehäusemantels 4 einer baugleichen Luer-Säule 2 reibschlüssig eingesteckt werden. Der Innendurchmesser a1 des ersten Endabschnitts 11 des Gehäusemantels 4 ist dazu etwas größer als der Außendurchmesser a2 des zweiten Endabschnitts 13 des Gehäusemantels 4, d.h. a1 > a2.

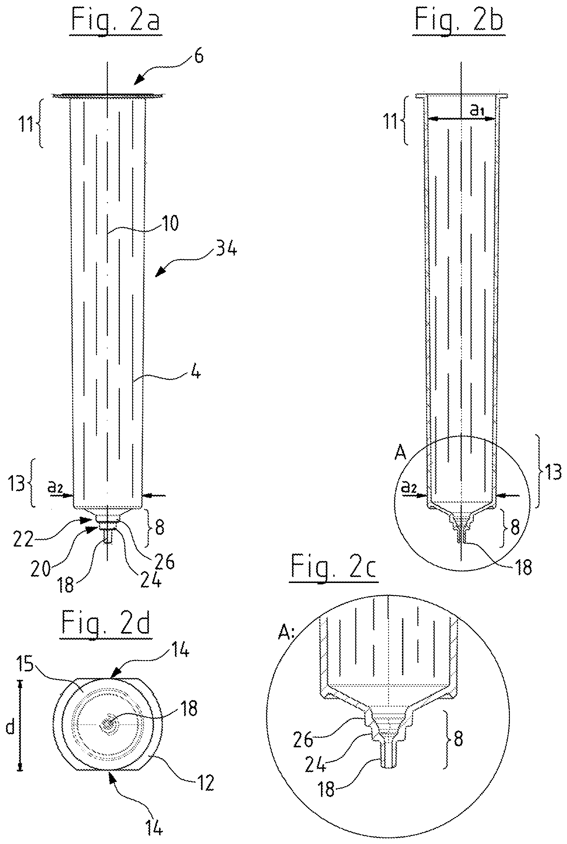

Das Einlassende 6 weist einen radialen Vorsprung 12 auf, der zum Halten der Luer-Säule 2 oder als Anschlag bei der Aufnahme der Luer-Säule in einem Halter geeignet ist. Der Vorsprung 12 basiert exemplarisch auf einer flachen Ringscheibe, von der zwei einander gegenüberliegende Ringsegmente entfernt sind. Zwei einander gegenüberliegende parallele Begrenzungskanten 14 werden dadurch gebildet. Der Abstand d der Begrenzungskanten 14 zueinander ist dabei größer als der Außendurchmesser des Gehäusemantels 4 am Einlassende 6. Dadurch wird, wie in Fig. 2d gezeigt, vorzugsweise ein vollständig umlaufender Flansch 15 mit einem Außendurchmesser gebildet, der dem Abstand d der Begrenzungskanten 14 zueinander entspricht. Auf dem Flansch 15 kann beispielsweise eine abziehbare Versiegelungsfolie sicher aufgebracht werden.

In dem Auslassabschnitt 8 ist an einem distalen Ende 16 der Luer-Säule 2 ein männlicher Luer-Kegel 18 angeordnet. Dieser weist beispielhaft eine Länge L1 von höchstens 7,5 mm auf und ist dazu angepasst, mit einem weiblichen Luer-Innenkegel eine Verbindung einzugehen. In einem verbleibenden Übergangsbereich 9 des Auslassabschnitts 8 sind zwei Durchmesserstufen 20 und 22 vorgesehen, die jeweils eine radiale Ringfläche 24 bzw. 26 bilden und eine axiale Länge L2 bzw. L3 aufweisen. Die axialen Längen L2 und L3 der ersten und zweiten Durchmesserstufen 20 und 22 sind bevorzugt identisch. Kanten 28 und 30 zwischen dem Luer-Kegel 18, der ersten Durchmesserstufe 20 und der zweiten Durchmesserstufe 22 sind abgerundet oder abgeschrägt. Im Innern des Auslassabschnitts 8 wird ein weitgehend kontinuierlicher Übergang zwischen den Innendurchmessern außerhalb des Auslassabschnitts 8 bis zu dem distalen Ende 16 geschaffen.

Beispielhaft weist die erste Durchmesserstufe 20 einen kleinsten Außendurchmesser von 8,9 mm auf. Die zweite Durchmesserstufe 22 weist beispielhaft einen kleinsten Außendurchmesser von 12,8 mm auf. Der Luer-Kegel weist weiterhin einen kleinsten Durchmesser von 4,4 mm auf. Die Durchmesserstufen 20 und 22 werden an einer von dem Luer-Kegel 18 abgewandten Seite durch einen Absatz begrenzt. Beide Durchmesserstufen 20 und 22 laufen zu dem distalen Ende 16 hin konisch zu. Der Kegelwinkel ist dabei mit dem des Luer-Kegels 18 vergleichbar oder identisch. Die Durchmesserstufen 20 und 22 sowie der Luer-Kegel weisen folglich nur weitgehend konstante Durchmesser auf.

Die Luer-Säule 2 kann ein Filterelement 32 aufnehmen (gestrichelt in Figur 1b), welches von Fluid durchströmt wird, das durch das Einlassende 6 in die Luer-Säule 2 gerät.

Die Figuren 2a bis 2d zeigen eine Luer-Säule 34, bei der der Gehäusemantel 4 einen größeren Außendurchmesser bildet. Der Auslassabschnitt 8 ist jedoch derselbe wie in den vorherigen Figuren. Die Luer-Säule 34 kann beispielhaft mittels der zweiten Durchmesserstufe 22 in das Einlassende 6 der Luer-Säule 2 aus den vorherigen Figuren gesteckt werden. Es ist vorstellbar, Luer-Säulen mit weiteren Durchmessern an dem Einlassende 6 bereitzustellen. Neben etwa 13 mm (Figuren 1a bis 1d) und etwa 37 mm (Figuren 2a bis 2d) sind auch andere Durchmesser denkbar.

### Bezugszeichenliste

- 2: Luer-Säule
- 4: Gehäusemantel
- 6: Einlassende
- 8: Auslassabschnitt
- 9: Übergangsbereich
- 10: Mittelachse
- 11: erster Endabschnitt des Gehäusemantels
- 12: radialer Vorsprung
- 13: zweiter Endabschnitt des Gehäusemantels
- 14: Begrenzungskante
- 16: distales Ende
- 18: Luer-Kegel
- 20: erste Durchmesserstufe
- 22: zweite Durchmesserstufe
- 24: erste radiale Ringfläche
- 26: zweite radiale Ringfläche
- 28: Kante
- 30: Kante
- 32: Filterelement
- 34: Luer-Säule

- L1: Länge Luer-Kegel
- L2: Länge erste Durchmesserstufe
- L3: Länge zweite Durchmesserstufe
- a1: Innendurchmesser des ersten Endabschnitts des Gehäusemantels
- a2: Außendurchmesser des zweiten Endabschnitts des Gehäusemantels
- d: Abstand der Begrenzungskanten zueinander

## Patentansprüche

1. Luer-Säule (2, 34) mit einem Einlassende (6), einem davon beabstandeten Auslassabschnitt (8) und einem sich von dem Einlassende (6) zu dem Auslassabschnitt (8) erstreckenden Gehäusemantel (4), wobei sich der Auslassabschnitt (8) in eine von dem Einlassende (6) abgewandte Richtung erstreckt und an einem distalen Ende (16) einen männlichen Luer-Kegel (18) ausbildet, wobei ein Übergangsbereich zwischen dem Luer-Kegel (18) und dem Gehäusemantel (4) einen abgestuften Durchmesser mit mindestens zwei Durchmesserstufen (20, 22) mit jeweils einer radialen Ringfläche (24, 26) aufweist, wobei die Luer-Säule (2, 34) einstückig ausgeführt ist, wobei der Gehäusemantel (4) in einem ersten am Einlassende (6) angeordneten Endabschnitt (11) des Gehäusemantels (4) und in einem zweiten vom Einlassende (6) abgewandten Endabschnitt (13) des Gehäusemantels (4) konisch zuläuft, sodass der zweite Endabschnitt (13) des Gehäusemantels (4) in einen ersten Endabschnitt (11) des Gehäusemantels (4) einer gleichartigen Luer-Säule (2, 34) reibschlüssig einsteckbar ist.

2. Luer-Säule (2, 34) nach Anspruch 1, wobei die radialen Ringflächen (24, 26) der Durchmesserstufen jeweils dieselbe axiale Länge (L2, L3) aufweisen.

3. Luer-Säule (2, 34) nach Anspruch 1 oder 2, wobei die radialen Ringflächen (24, 26) an ihren axialen Endkanten abgerundet oder abgeschrägt sind.

4. Luer-Säule (2, 34) nach einem der vorhergehenden Ansprüche, wobei die radiale Ringfläche (24, 26) eine axiale Länge (L2, L3) von höchstens 7,5 mm, bevorzugt von 3 bis 6 mm und besonders bevorzugt von 4 mm aufweist.

5. Luer-Säule (2, 34) nach Anspruch 1, wobei sich der erste Endabschnitt (11) des Gehäusemantels (4) und/oder der zweite Endabschnitt (13) des Gehäusemantels (4) über 1% bis 5% der Länge des Gehäusemantels (4) erstreckt.

6. Luer-Säule (2, 34) nach Anspruch 1, wobei der Gehäusemantel (4) über seine gesamte Länge konisch zuläuft.

7. Luer-Säulen-System mit mindestens zwei Luer-Säulen (2, 34) nach einem der vorhergehenden Ansprüche, wobei ein Außendurchmesser einer der mindestens einen Durchmesserstufe (20, 22) einer der Luer-Säulen (2, 34) angepasst ist, eine Klemmpassung zwischen der betreffenden radialen Ringfläche (24, 26) und dem Einlassende (6) der anderen Luer-Säule (2, 34) zu bilden.

## Claims

1. Luer column (2, 34) with an inlet end (6), an outlet section (8) at a distance from the inlet end (6), and a housing shell (4) that extends from the inlet end (6) to the outlet section (8), wherein the outlet section (8) extends in a direction facing away from the inlet end (6) and forms a male Luer cone (18) at a distal end (16), wherein a transition area between the Luer cone (18) and the housing shell (4) has a stepped diameter with at least two diameter steps (20, 22) respectively with an annular radial surface (24, 26), wherein the Luer column (2, 34) is configured as one piece, wherein the housing shell (4) tapers conically in a first end section (11) of the housing shell (4) arranged at the inlet end (6) and in a second end section (13) of the housing shell (4) facing away from the inlet end (6), so that the second end section (13) of the housing shell (4) can be inserted in a frictionally engaged manner into a first end section (11) of the housing shell (4) of a similar Luer column (2, 34).

2. Luer column (2, 34) according to claim 1, wherein the annular radial surfaces (24, 26) of the diameter steps respectively have the same axial length (L2, L3).

3. Luer column (2, 34) according to claim 1 or 2, wherein the annular radial surfaces (24, 26) are rounded or bevelled at their axial end edges.

4. Luer column (2, 34) according to one of the preceding claims, wherein the annular radial surface (24, 26) has an axial length (L2, L3) of at most 7.5 mm, preferably of 3 to 6 mm and particularly preferably of 4 mm.

5. Luer column (2, 34) according to claim 1, wherein the first end section (11) of the housing shell (4) and/or the second end section (13) of the housing shell (4) extends over 1% to 5% of the length of the housing shell (4).

6. Luer column (2, 34) according to claim 1, wherein the housing shell (4) is conically tapered over its entire length.

7. Luer column system with at least two Luer columns (2, 34) according to any one of the preceding claims, wherein an outer diameter of one of the at least one diameter steps (20, 22) of one of the Luer columns (2, 34) is adapted to form an interference fit between the relevant annular radial surface (24, 26) and the inlet end (6) of the other Luer column (2, 34).

## Revendications

1. Colonne Luer (2, 34) comportant une extrémité d'entrée (6), une extrémité de sortie (8) qui en est écartée, et une enveloppe de corps (4) s'étendant depuis l'extrémité d'entrée (6) vers l'extrémité de sortie (8), l'extrémité de sortie (8) s'étendant dans un sens opposé à l'extrémité d'entrée (6) et formant un cône mâle Luer (18) à son extrémité distale (16), une zone de transition entre le cône Luer (18) et l'enveloppe de corps (4) ayant un diamètre étagé comportant au moins deux étages de diamètre (20, 22) dont chacun comporte une face annulaire radiale (24, 26), la colonne Luer (2, 34) étant réalisée sous une forme monobloc, l'enveloppe de corps (4) s'amenuisant, dans une première partie terminale (11) de l'enveloppe de corps (4) disposée à l'extrémité d'entrée (6) et dans une seconde partie terminale (13) de l'enveloppe de corps (4) située à l'opposé de l'extrémité d'entrée (6), de manière à former un cône, faisant en sorte que la seconde partie terminale (13) de l'enveloppe de corps (4) puisse s'emboîter dans la première partie terminale (11) de l'enveloppe de corps (4) d'une colonne Luer (2, 34) du même type pour ainsi établir une liaison par friction.

2. Colonne Luer (2, 34) selon la revendication 1, les faces annulaires radiales (24, 26) des étages de diamètre présentant chacune la même longueur axiale (L2, L3).

3. Colonne Luer (2, 34) selon les revendications 1 ou 2, les bords terminaux axiaux des faces annulaires radiales (24, 26) étant arrondis ou chanfreinés.

4. Colonne Luer (2, 34) selon l'une des revendications précédentes, la face annulaire radiale (24, 26) ayant une longueur axiale (L2, L3) inférieure ou égale à 7,5 mm, de préférence comprise entre 3 et 6 mm et, avec une préférence particulière, égale à 4 mm.

5. Colonne Luer (2, 34) selon la revendication 1, la première partie terminale (11) de l'enveloppe de corps (4) et/ou la seconde partie terminale (13) de l'enveloppe de corps (4) s'étendant sur 1% à 5% de la longueur de l'enveloppe de corps (4).

6. Colonne Luer (2, 34) selon la revendication 1, l'enveloppe de corps (4) s'amenuisant sur toute sa longueur de manière à former un cône.

7. Système de colonnes Luer comportant au moins deux colonnes Luer (2, 34) selon l'une des revendications précédentes, un diamètre extérieur de l'un des étages de diamètre (20, 22), dont l'une des colonnes Luer (2, 34) présente au moins un, étant adapté de manière à ce que la face annulaire radiale (24, 26) concernée puisse épouser l'extrémité d'entrée (6) de l'autre colonne (2, 34) par serrage.
